# EUROPÄISCHE PATENTSCHRIFT

(11) **EP 2 443 420 B1**
(45) Veröffentlichungstag und Bekanntmachung des Hinweises auf die Patenterteilung: **10.02.2016**
(21) Anmeldenummer: 10739293.8
(22) Anmeldetag: 07.05.2010
(51) Int. Cl.: G01D 11/30

(54) **BEHÄLTER MIT EINEM SENSORADAPTER**
CONTAINER WITH A SENSOR ADAPTER
CONTENANT DOTÉ D'UN ADAPTATEUR POUR DÉTECTEUR

(30) Priorität: 16.06.2009 DE 102009025419; 14.08.2009 DE 102009037345
(43) Veröffentlichungstag der Anmeldung: 25.04.2012
(73) Patentinhaber: Sartorius Stedim Biotech GmbH, 37079 Göttingen (DE)
(72) Erfinder: BAUMFALK, Reinhard, 37083 Göttingen (DE); KAHLERT, Wolfgang, 34327 Körle (DE); GRELLER, Gerhard, 37085 Göttingen (DE)
(86) Internationale Anmeldenummer: PCT/EP2010/002808
(87) Internationale Veröffentlichungsnummer: WO 2010/145735

(56) Entgegenhaltungen:
- WO-A1-2008/071022
- DE-A1-102009 052 266
- US-A- 6 131 473
- US-A1- 2005 042 133

## Beschreibung

Die Erfindung betrifft einen Behälter mit einem Sensoradapter für die Aufnahme einer Sensoranordnung zur Messung mindestens eines Parameters von in einem Behälterinnenraum enthaltenen Medien, wobei der Sensoradapter an der Wandung zum Behälterinnenraum angeordnet ist und eine von außen zugängliche Aufnahmeöffnung eines zum Behälterinnenraum hin umgrenzten Aufnahmekanals zur Adaption der Sensoranordnung aufweist.

### Stand der Technik

Aus der DE 10 2006 001 610 B4 ist ein Bioreaktor bzw. Behälter bekannt, der insbesondere als Einwegmischbeutel ausgebildet ist und einen Sensoradapter zur Aufnahme einer Sensoranordnung in Form eines optischen Sensors, der von einem Ende eines Lichtleiters gebildet wird und reversibel angebracht ist, aufweist. Der in der Wandung zum Reaktorinnenraum angeordnete Sensoradapter weist eine von außen zugängliche Aufnahmeöffnung eines zum Reaktorinnenraum hin von einem transparenten Material umgrenzten Aufnahmekanals auf.

Nachteilig bei dem bekannten Sensoradapter ist, dass er lediglich für optische Messvorgänge geeignet ist. Zum Einbau von klassischen reversiblen Elektroden bzw. Sensoranordnungen zur Messung des pH-Wertes, des Gehaltes an Kohlendioxid oder des Sauerstoffs oder zur Messung der Leitfähigkeit ist der Sensoradapter nicht geeignet. Auch sind die bekannten Adapter nicht zur Verwendung von Gassensoren, wie sie für gelösten Sauerstoff oder gelöstes Kohlendioxid bekannt sind, geeignet.

Weiterhin sind Sensoradapter aus der DE 10 2006 022 307 A1 und aus der WO 2006/017951 A1 bekannt, die ebenfalls die oben genannten Nachteile aufweisen.

In WO 2008/071022 A1 wird ein Adapter für Drucksensoren beschrieben, bei dem der Gasdruck in einem Verbrennungsmotor durch Schwingen (Verformung) einer dichten Sensormembran von einem Sensor detektiert wird. Dabei kommt der Sensor selbst nicht mit dem zu messenden Gas in Kontakt.

Bei dem in US 6 131 473 A beschriebenen Sensoradapter befinden sich benachbart zu dem geschlossenen Ende eines rohrförmigen Adapterschaftes schlitzförmige Öffnungen, über welche das zu messende Medium mit dem in diesem Bereich befindlichen Sensor in Kontakt gelangt. Wenn nicht gemessen werden soll, kann der Adapterschaft mit seinem geschlossenen Ende aus dem Behälter soweit heraus gefahren werden, dass die offenen Schlitze nicht mehr vom Medium durchdrungen werden.

In US 2005/0042133 A1 wird ein chemischer Analysesensor beschrieben, bei dem abgedichtet durch die Wand eines Behälters mit einem Prozessfluid ein poröser rohrförmiger Filter ragt zur Abtrennung eines zu messenden Mediums, das in einen vom rohrförmigen Filter gebildeten Hohlraum eindringt. Hier kommt das zu messende Medium mit einem Sensor in Kontakt. Der poröse rohrförmige Filter ist innen oder außen mit einer chemisch selektiv wirkenden Beschichtung überzogen. Diese Beschichtung erlaubt es, einer ersten chemischen Substanz aus dem Prozessfluid in das zu messende Medium zu diffundieren, während andere chemische Substanzen durch die Beschichtung von einem Eindringen in das zu messende Medium ausgeschlossen werden.Nachteilig ist die lange Ansprechzeit, weil eine Diffusion von Substanzen durch die Beschichtung deutlich langsamer verläuft als eine konvektive Strömung durch einen porösen Filter allein. Weitere Nachteile sind die Abgabe von Stoffen aus der chemisch selektiv wirkenden Beschichtung in das Prozessfluid, was insbesondere in biotechnologischen oder pharmazeutischen Prozessen schädlich sein kann, und die Verfälschung der Messungen, weil sich die Zusammensetzung des zu messenden Mediums in dem Hohlraum durch den Ausschluss von Substanzen von der Zusammensetzung des Prozessfluids unterscheidet.

Sollen insbesondere Gassensoren zum Einsatz kommen, müssen die bekannten Adapter entweder fest eingebaute Sensoren nutzen, die zum einen nicht reversibel sind und die zum anderen anfällig gegen eine notwendige Sterilisation sind oder die Adapter müssen zum Reaktorinnenraum hin geöffnet sein. Soweit die Adapter zum Reaktorinnenraum hin geöffnet sind, können zwar austauschbare Gassensoren verwendet werden, diese müssen dann aber ebenfalls sterilisiert werden, was mit Problemen verbunden ist.

Unter Behältern werden im Rahmen der vorliegenden Erfindung insbesondere Behälter zum Mischen, Lagern und Transportieren, sowie Bioreaktoren und Fermenter verstanden. Die Behälter können insbesondere aus Kunststoff ausgebildet sein.

Als Medien werden im Rahmen der vorliegenden Erfindung insbesondere Flüssigkeiten, Gase, Suspensionen, Dispersionen, Puffer und Zellkulturbrühen angesehen.

Unter Parametern werden im folgenden insbesondere Konzentration von Stoffen, Druck und Partialdruck von Gasen (Sauerstoff, Kohlendioxid), Feuchtigkeit, Anzahl Partikel, Trübung, Temperatur, pH-Wert, elektromagnetische Strahlen, Fluoreszenz, elektrische Leitfähigkeit, sowie kapazitive und elektrische Widerstände verstanden.

### Aufgabenstellung

Aufgabe der vorliegenden Erfindung ist es daher, die bekannten Behälter mit einem Sensoradapter derart weiterzubilden, dass Sensoranordnungen eingesetzt werden können, die unter Aufrechterhaltung der Sterilität Kontakt mit den zu messenden Medien benötigen, wie beispielsweise Gassensoren.

### Darstellung der Erfindung

Diese Aufgabe wird in Verbindung mit den Merkmalen des Oberbegriffs von Anspruch 1 dadurch gelöst, dass der Aufnahmekanal des Sensoradapters zum Behälterinnenraum hin mindestens eine von einer Membran gebildete Grenzfläche aufweist, über die der Sensoranordnung das oder die zu messenden Medien zuführbar sind. Die Membranen weisen eine Porengröße zwischen 0,1 und 0,4 µm, bevorzugt von ≤ 0,2 µm auf. Damit bilden die Membranen eine Sterilbarriere zwischen Reaktorinnenraum und der reversibel anbringbaren Sensoranordnung.

Durch die Ausbildung jedenfalls eines Teiles der Grenzfläche zum Reaktorinnenraum als Membran, wird es möglich, dass das zu messendes Medium mit den Sensoren der Sensoranordnung direkt in Kontakt kommt. Gleichzeitig wird durch die Membran vermieden, dass der Reaktorinnenraum von der Sensoranordnung bzw. deren Sensoren kontaminiert wird. Die Sensoranordnung mit empfindlichen Sensoren muss nicht sterilisiert werden sondern lediglich der am Bioreaktor befestigte Sensoradapter.

Nach einer weiteren bevorzugten Ausführungsform der Erfindung weist die Membran in Abhängigkeit vom kontaktierten Medium hydrophile, hydrophobe oder oleophobe Eigenschaften auf. Je nach Ausbildung des Sensoradapters kann beispielsweise die erste Membran in das im Reaktorinnenraum angeordnete flüssige Medium eintauchen, während beispielsweise die zweite Membran im Kopfraum des Reaktorinnenraums angeordnet ist und Kontakt mit der Gasphase aufweist. Je nach kontaktiertem Medium ist es dann zweckmäßig, die Membran hydrophil oder hydrophob auszubilden. Gleichwohl sollte der Sensoradapter relativ keimarm sein.

Nach einer weiteren bevorzugten Ausführungsform der Erfindung weist die Membran einen oder mehrere Bereiche mit hydrophilen Eigenschaften und einen oder mehrere Bereiche mit hydrophoben Eigenschaften auf. Beispielsweise ist die Membran als eine hydrophile Membran mit einer Mehrzahl von hydrophoben Spots oder als eine hydrophobe Membran mit einer Mehrzahl von hydrophilen Spots ausgebildet. So kann bei einem Vorschub der Sensoranordnung über die hydrophoben Bereiche der Membran Gas steril in den Behälterinnenraum gedrückt werden. Flüssiges Medium aus dem Behälterinnenraum kann die Membran über die hydrophilen Bereiche durchdringen und den Sensor der Sensoranordnung umspülen.

Gemäß einer weiteren bevorzugten Ausführungsform der Erfindung ist die Membran aus einem natürlichen Polymer, wie z.B. Cellulose-Acetat oder regenerierter Cellulose ausgebildet.

Nach einer weiteren Ausführungsform der Erfindung ist die Membran aus einem synthetischen Polymer, wie beispielsweise Polysulfon ausgebildet.

Gemäß einer weiteren bevorzugten Ausführungsform der Erfindung bildet der Aufnahmekanal einen in den Behälterinnenraum hineinragenden Adapterschaft, der zwei hintereinander angeordnete Membranen unterschiedlicher Eigenschaften aufweist und dessen Stirnfläche verschlossen ist.

Nach einer weiteren bevorzugten Ausführungsform der Erfindung bildet der Aufnahmekanal einen in den Behälterinnenraum hineinragenden Adapterschaft, der eine erste seine Stirnfläche verschließende Membran und eine zweite auf seinem Umfang angeordnete Membran mit gegenüber der ersten Membran unterschiedlichen Eigenschaften aufweist. Der Aufnahmekanal kann aber auch einen in den Behälterinnenraum hineinragenden Adapterschaft bilden, der eine auf seinem Umfang angeordnete erste Membran aufweist, dessen Stirnfläche verschlossen ist und der einen nach außen führenden Seitenkanal aufweist, dessen außerhalb des Reaktorinnenraumes liegende Zugangsöffnung ebenfalls von einer Membran oder einem eine Membran aufweisenden Filterelement abgedeckt ist. Die zweite Membran kann dabei ebenfalls hydrophile, hydrophobe oder andere Eigenschaften aufweisen. Der nach außen führende Seitenkanal ist insbesondere zur Gewinnung von zellfreier Flüssigkeit geeignet, die einem Analysengerät zugeführt werden kann.

Nach einer weiteren bevorzugten Ausführungsform der Erfindung ist die Sensoranordnung kolbenförmig in dem Aufnahmekanal zur Erzeugung eines Druckes längsverschieblich angeordnet. Die Längsverschieblichkeit kann dabei beispielsweise durch einen Faltenbalg erreicht werden, über den die im Aufnahmekanal angeordnete Sensoranordnung mit dem Sensoradapter verbunden ist.

Gemäß einer weiteren bevorzugten Ausführungsform der Erfindung ist der Sensoradapter aus einem gegen Betastrahlung und/oder Gammastrahlung und/oder gegen Ethylenoxid (ETO) resistenten Material ausgebildet. Damit ist der Sensoradapter geeignet zusammen mit dem Einwegbiorektor sterilisiert zu werden.

Der Sensoradapter kann beispielsweise in einer Seitenwandung oder auch im Kopfbereich des Behälters angeordnet werden. So ist sein Einbau in Höhe der im Reaktorinnenraum befindlichen Flüssigkeit möglich, aber auch ein Einbau im Kopfraum. Die Membran kann Kontakt mit den flüssigen Medien haben, sie kann aber auch Kontakt mit der Gasphase im Kopfraum haben. Selbstverständlich muss der Sensoradapter an die vorgesehene Sensoranordnung jeweils angepasst sein.

Gemäß einer weiteren bevorzugten Ausführungsform der Erfindung ist der Behälter als ein Einwegbioreaktor ausgebildet.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:
- Figur 1:: eine schematische Seitenansicht eines Behälters im Ausriss mit einem Sensoradapter im Schnitt und einer ungeschnittenen Sensoranordnung,
- Figur 2:: eine Seitenansicht im Schnitt und Ausriss eines weiteren Behälters mit einem Sensoradapter,
- Figur 3:: eine Seitenansicht im Schnitt und Ausriss eines weiteren Behälters mit einem Sensoradapter und nach außen führendem Seitenkanal,
- Figur 4:: eine Seitenansicht im Schnitt und Ausriss eines weiteren Behälters mit einem Sensoradapter und einer Sensoranordnung und
- Figur 5:: eine Seitenansicht im Schnitt und Ausriss eines weiteren Behälters mit einem Sensoradapter, nach außen führendem Seitenkanal und einer Membran mit unterschiedlichen Bereichen

### Beschreibung der Ausführungsbeispiele

Ein Behälter 2, der im Ausführungsbeispiel als Einwegbioreaktor 1 ausgebildet ist, besteht im Wesentlichen aus dem Behälter 2 und einem Sensoradapter 3.

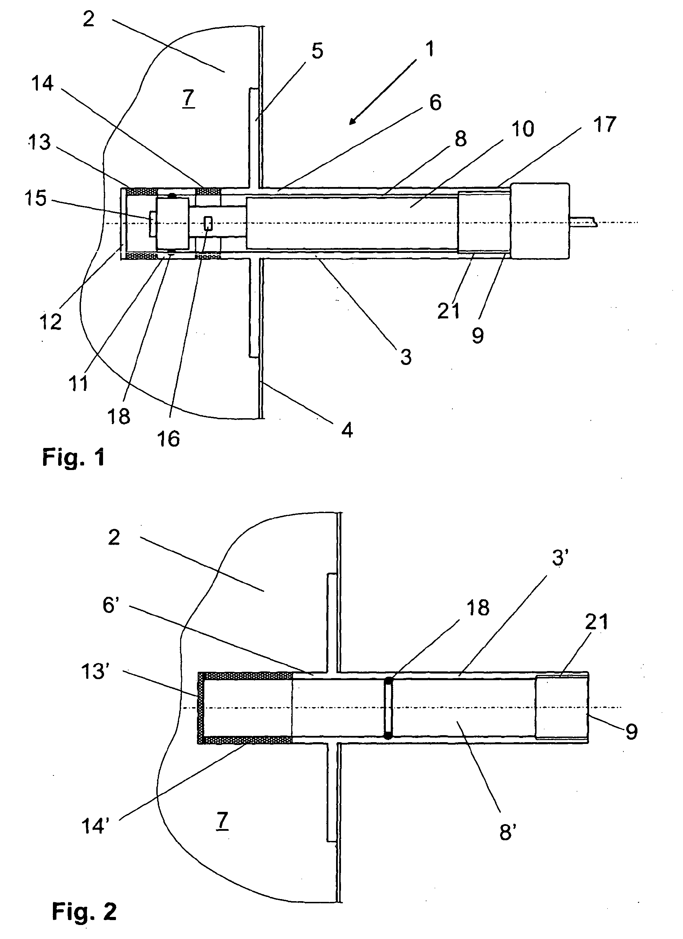

Der Behälter 2 ist im Ausführungsbeispiel der Figur 1 als ein faltbarer flexibler Beutel ausgebildet, in dessen Wandung 4 der Sensoradapter 3 angeordnet ist. Der Sensoradapter 3 weist einen Flansch 5 auf, der mit der Wandung 4 verschweißt ist. Der Sensoradapter 3 weist einen Adapterschaft 6 auf, der von außerhalb des Behälters 2 durch die Wandung 4 hindurch in den Reaktorinnenraum 7 hineinragt. Zentral weist der Sensoradapter 3 bzw. dessen Adapterschaft 6 einen Aufnahmekanal 8 mit einer von außen zugänglichen Aufnahmeöffnung 9 auf. Über die Aufnahmeöffnung 9 ist eine Sensoranordnung zur Messung eines Parameters von in dem Reaktorinnenraum 7 enthaltenen Medien in den Aufnahmekanal 8 einführbar und reversibel mit dem Sensoradapter 3 verbindbar.

Entsprechend den Ausführungsformen der Figuren 1 bis 3 ragt der Adapterschaft 3 mit seinem behälterseitigen Ende 11 in den Reaktorinnenraum 7 hinein.

Entsprechend dem Ausführungsbeispiel von Figur 1 ist das behälterseitige Ende 11 des Adapterschaftes 6 an seiner Stirnfläche durch eine Abdeckung 12, die transparent ausgebildet sein kann, verschlossen. Zur Abdeckung 12 hin, weist der Adapterschaft 6 bzw. der Aufnahmekanal 8 eine erste Membran 13 auf. Neben der ersten Membran 13 in Richtung Flansch 5 weist der Aufnahmekanal 8 bzw. das behälterseitige Ende 11 des Adapterschaftes 6 eine zweite Membran 14 auf. Die aus einem Polymer ausgebildeten Membranen 13, 14 weisen eine Porengröße von ≤ 0,2 µm auf, um zwischen den Reaktorinnenraum 7 und dem Aufnahmekanal 8 eine Sterilbarriere zu bilden. Die in dem Aufnahmekanal 8 eingesetzte Sensoranordnung 10 weist den Membranen 13, 14 zugeordnete Sensoren 15, 16 auf. An seinem die Aufnahmeöffnung 9 aufweisenden Ende 17 weist der Aufnahmekanal 8 bzw. der Adapterschaft 6 ein Gewinde 18 zur Befestigung der Sensoranordnung 10 auf. Die Wandung des Aufnahmekanals 8 weist einen Dichtring 18 zur Abdichtung und Führung der Sensoranordnung 10 auf.

Entsprechend dem Ausführungsbeispiel der Figur 2 weist der Adapterschaft 6' des Sensoradapters 3' an seiner dem Reaktorinnenraum 7 zugewandten Stirnseite die erste Membran 13' und benachbart am Umfang des Aufnahmekanals 8 die zweite Membran 14' auf.

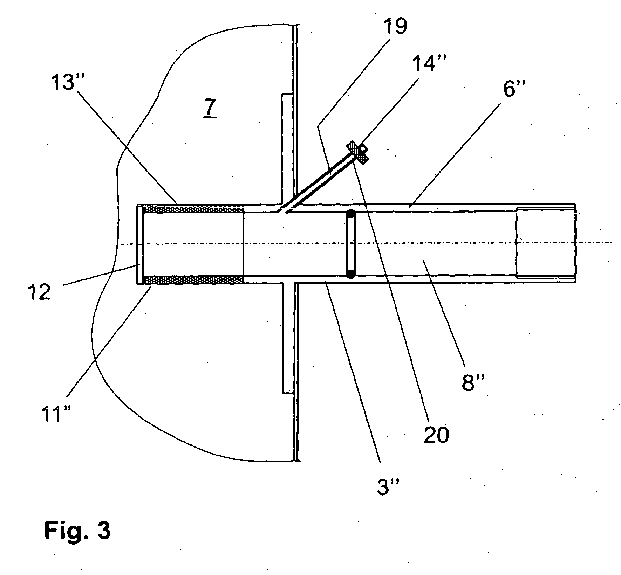

Nach dem Ausführungsbeispiel der Figur 3 weist der Adapterschaft 6" des Sensoradapters 3" an seinem behälterseitigen Ende 11" die erste Membran 13" auf. An seinem stirnseitigen Ende ist der Aufnahmekanal 8" von der Abdeckung 12 verschlossen. Der Adapterschaft 6" weist vom Aufnahmekanal 8" einen nach außen führenden Seitenkanal 19 auf, dessen außerhalb des Behälterinnenraumes liegende Zugangsöffnung 20 von einer zweiten Membran 14" abgedeckt ist.

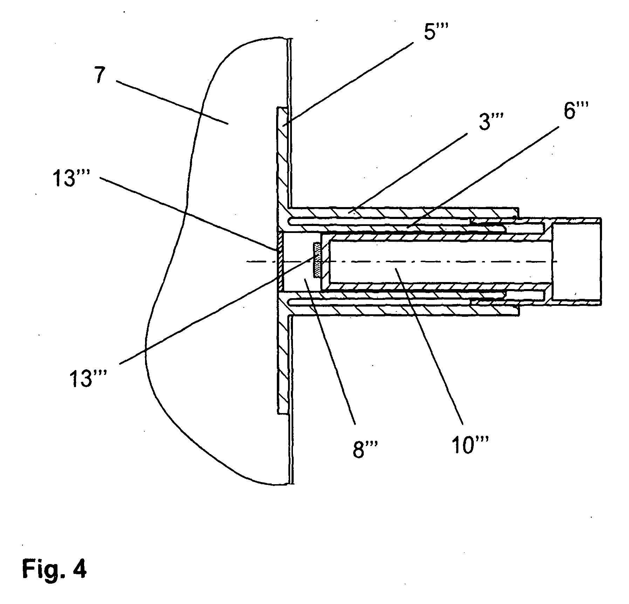

Gemäß der Ausführungsform von Figur 4 endet der Aufnahmekanal 8"' des Sensoradapters 3"' zum Reaktorinnenraum 7 hin in den Flansch 5"', wobei seine behälterseitige Öffnung von einer ersten Membran 13"' abgedeckt ist. In dem Aufnahmekanal 3"' wird die Sensoranordnung 10"' kolbenförmig geführt und ist längsverschieblich angeordnet.

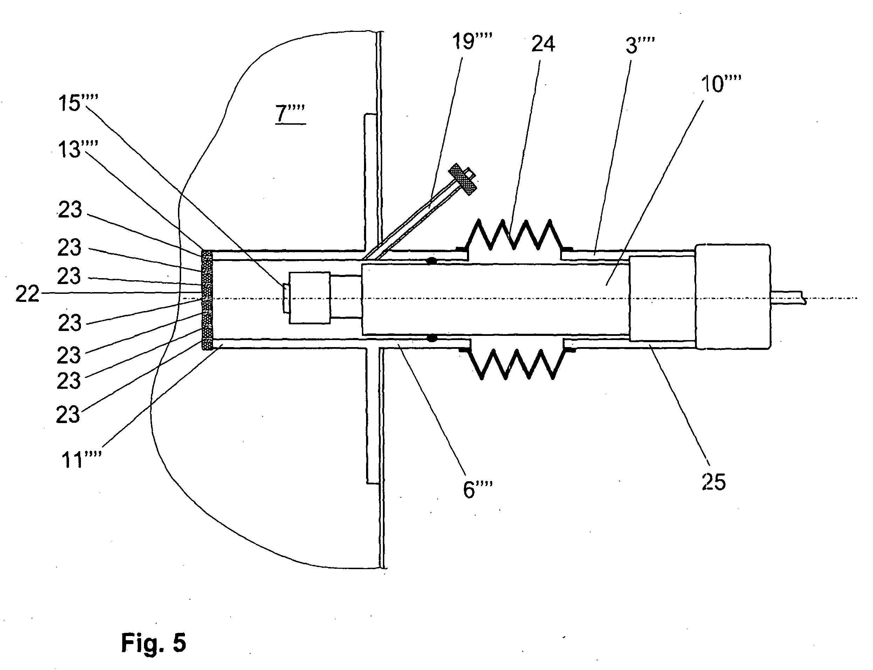

Nach dem Ausführungsbeispiel der Figur 5 weist der Adapterschaft 6"" des Sensoradapters 3"" an seinem behälterseitigen Ende 11"" die Membran 13"" auf. Die Membran 13"" weist einen Bereich 22 mit hydrophilen Eigenschaften auf, der von Spots bzw. Bereichen 23, die kleine Inseln bilden und hydrophobe Eigenschaften aufweisen, durchsetzt ist.

An seinem der Membran 13"" abgewandten Ende weist der Aufnahmekanal 8"" einen Adapteransatz 25 zur Ankupplung der Sensoranordnung 10"" auf. Der Adapteransatz 25 ist über einen Faltenbalg 24 mit dem Adapterschaft 6"" verbunden, sodass die Sensoranordnung 10"" in dem Aufnahmekanal 8"" des Adapterschaftes 6"" in Längsrichtung verschiebbar ist. Der Adapterschaft 6"" weist vom Aufnahmekanal 8"" einen nach außen führenden Seitenkanal 19"" auf, dessen außerhalb des Behälterinnenraumes 7"" liegende Zugangsöffnung 20"" von einer zweiten Membran 14"" abgedeckt ist.

Bei einem Vorschub der Sensoranordnung 10"" in Richtung Membran 13"" kann über den Seitenkanal 19"" aufgenommenes Gas über die hydrophoben Bereiche 23 steril in den Behälterinnenraum 7"" gedrückt werden. Hydrophiles Medium aus dem Behälterinnenraum 7"" kann in den Aufnahmekanal 8"" eindringen und den Sensor 15"" der Sensoranordnung 10"" umspülen.

## Patentansprüche

1. Behälter (2) mit einem Sensoradapter (3) für die Aufnahme einer Sensoranordnung (10, 10"', 10"") zur Messung mindestens eines Parameters von in einem Behälterinnenraum (7) enthaltenen Medien, wobei der Sensoradapter (3, 3', 3", 3"') an der Wandung zum Behälterinnenraum (7) angeordnet ist und eine von außen zugängliche Aufnahmeöffnung (9) eines zum Behälterinnenraum (7) hin umgrenzten Aufnahmekanals (8, 8", 8"') zur Adaption der Sensoranordnung (10, 10"', 10"") aufweist,
**dadurch gekennzeichnet,**
**dass** der Aufnahmekanal (8, 8", 8"') des Sensoradapters (10) zum Behälterinnenraum (7) hin mindestens eine von einer Membran (13, 13', 13", 13"', 13"", 14, 14', 14") gebildete Grenzfläche aufweist, über die der Sensoranordnung (10, 10"', 10"") das zu messende Medium oder die zu messenden Medien zuführbar sind, wobei die Membran (13, 13', 13", 13"', 13"", 14, 14', 14") eine Porengröße zwischen 0,1 und 0,4 µm aufweist.

2. Behälter nach Anspruch 1,
**dadurch gekennzeichnet,**
**dass** die Membran (13, 13', 13", 13"', 13"', 14, 14', 14") eine Porengröße von ≤ 0,2 µm aufweist und eine Sterilbarriere bildet.

3. Behälter nach einem der Ansprüche 1 bis 2,
**dadurch gekennzeichnet,**
**dass** die Membran (13, 13', 13'', 13''', 14, 14', 14'') hydrophile oder hydrophobe oder oleophobe Eigenschaften aufweist.

4. Behälter nach einem der Ansprüche 1 bis 2,
**dadurch gekennzeichnet,**
**dass** die Membran (13"") einen oder mehrere Bereiche (22) mit hydrophilen Eigenschaften und einen oder mehrere Bereiche (23) mit hydrophoben Eigenschaften aufweist.

5. Behälter nach einem der Ansprüche 1 bis 4,
**dadurch gekennzeichnet,**
**dass** die Membran (13, 13', 13", 13"', 13"", 14, 14', 14") aus einem natürlichen Polymer ausgebildet ist.

6. Behälter nach Anspruch 5,
**dadurch gekennzeichnet,**
**dass** die Membran (13, 13', 13", 13"', 13"", 14, 14', 14") aus Cellulose-Acetat oder regenerierter Cellulose ausgebildet ist.

7. Behälter nach einem der Ansprüche 1 bis 4,
**dadurch gekennzeichnet,**
**dass** die Membran (13, 13', 13", 13"', 13"", 14, 14', 14") aus einem synthetischen Polymer ausgebildet ist.

8. Behälter nach Anspruch 7,
**dadurch gekennzeichnet,**
**dass** die Membran (13, 13', 13", 13"', 13"", 14, 14', 14") aus Polysulfon ausgebildet ist.

9. Behälter nach einem der Ansprüche 1 bis 8,
**dadurch gekennzeichnet,**
**dass** der Aufnahmekanal (8) einen in den Reaktorinnenraum (7) hineinragenden Adapterschaft (6) bildet, der zwei hintereinander angeordnete Membranen (13, 14) mit unterschiedlichen Eigenschaften aufweist und dessen Stirnfläche verschlossen ist.

10. Behälter einem der Ansprüche 1 bis 8,
**dadurch gekennzeichnet,**
**dass** der Aufnahmekanal (8') einen in den Reaktorinnenraum (7) hineinragenden Adapterschaft (6') bildet, der eine erste seine Stirnfläche verschließende Membran (13') und eine zweite auf seinem Umfang angeordnete Membran (14') mit gegenüber der ersten Membran (13') unterschiedlichen Eigenschaften aufweist.

11. Behälter nach einem der Ansprüche 1 bis 8,
**dadurch gekennzeichnet,**
**dass** der Aufnahmekanal (8") einen in den Reaktorinnenraum (7) hineinragenden Adapterschaft (6") bildet, der eine auf seinem Umfang angeordnete erste Membran (13") aufweist, dessen Stirnfläche verschlossen ist und der einen nach außen führenden Seitenkanal (19) aufweist, dessen außerhalb des Reaktorinnenraumes (7) liegende Zugangsöffnung (20) von einer zweiten Membran (14'') abgedeckt ist.

12. Behälter nach einem der Ansprüche 1 bis 11,
**dadurch gekennzeichnet,**
**dass** die Sensoranordnung (10") kolbenförmig in dem Aufnahmekanal (8"') zur Erzeugung eines Druckes längsverschieblich angeordnet ist.

13. Behälter nach einem der Ansprüche 1 bis 12,
**dadurch gekennzeichnet,**
**dass** die Sensoranordnung (10"" ) über einen Faltenbalg (24) längsverschieblich in dem Aufnahmekanal (8"") angeordnet ist.

14. Behälter nach einem der Ansprüche 1 bis 13,
**dadurch gekennzeichnet,**
**dass** der Sensoradapter (3, 3', 3", 3"', 3"") aus einem gegen Betastrahlung und/oder gegen Gammastrahlung und/oder gegen ETO resistenten Material besteht.

15. Behälter nach einem der Ansprüche 1 bis 14,
**dadurch gekennzeichnet,**
**dass** der Behälter (2) als ein Einwegbioreaktor (1) ausgebildet ist.

## Claims

1. Container (2) with a sensor adapter (3) for mounting a sensor arrangement (10, 10"', 10"") for measuring at least one parameter of media contained in a container interior space (7), wherein the sensor adapter (3, 3', 3", 3"') is arranged at the wall towards the container interior space (7) and has a mounting opening (9), which is accessible from outside, of a mounting channel (8, 8", 8"'), which is bounded towards to the container interior space (7), for adaptation of the sensor arrangement (10, 10"', 10""),
**characterised in that**
the mounting channel (8, 8", 8"') of the sensor adapter (10) has towards the container interior space (7) at least one boundary surface formed by a membrane (13, 13', 13", 13"', 13"", 14, 14', 14") by way of which the medium or media to be measured can be fed to the sensor arrangement (10, 10"', 10""), wherein the membrane (13, 13', 13", 13"', 13"", 14, 14', 14") has a pore size between 0.1 and 0.4 microns.

2. Container according to claim 1, **characterised in that** the membrane (13, 13', 13", 13"', 13"", 14, 14', 14") has a pore size of ≤ 0.2 microns and forms a sterile barrier.

3. Container according to one of claims 1 and 2, **characterised in that** the membrane (13, 13', 13", 13"', 14, 14', 14") has hydrophilic, hydrophobic or oleophobic properties.

4. Container according to one of claims 1 and 2, **characterised in that** the membrane (13"") has one or more regions (22) with hydrophilic properties and one or more regions (23) with hydrophobic properties.

5. Container according to any one of claims 1 to 4, **characterised in that** the membrane (13, 13', 13", 13"', 13"", 14, 14', 14") is formed from a natural polymer.

6. Container according to claim 5, **characterised in that** the membrane (13, 13', 13", 13"', 13"", 14, 14', 14") is formed from cellulose acetate or regenerated cellulose.

7. Container according to any one of claims 1 to 4, **characterised in that** the membrane (13, 13', 13", 13"', 13"", 14, 14', 14") is formed from a synthetic polymer.

8. Container according to claim 7, **characterised in that** the membrane (13, 13', 13", 13"', 13"", 14, 14', 14") is formed from polysulfone.

9. Container according to any one of claims 1 to 8, **characterised in that** the mounting channel (8) forms an adapter shaft (6) which protrudes into the reactor interior space (7) and comprises two membranes 13, 14) with different properties arranged one behind the other and the end surface of which is closed.

10. Container according to any one of claims 1 to 8, **characterised in that** the mounting channel (8') forms an adapter shaft (6') which protrudes into the reactor interior space (7) and which has a first membrane (13') closing its end surface and a second membrane (14') arranged on its circumference and having different properties in relation to the first membrane (13').

11. Container according to any one of claims 1 to 8, **characterised in that** the mounting channel (8") forms an adapter shaft (6"), which protrudes into the reactor interior space (7) and has a first membrane (13") arranged on its circumference and the end surface of which is closed, the membrane having a lateral channel (19) which leads outwardly and the access opening (20) of which lying outside the reactor interior space (7) is covered by a second membrane (14").

12. Container according to any one o claims 1 to 11, **characterised in that** the sensor arrangement (10") is arranged in piston shape in the mounting channel (8"') to be longitudinally displaceable for producing a pressure.

13. Container according to any one of claims 1 to 12, **characterised in that** the sensor arrangement (10"") is arranged in the mounting channel (8"") by way of a bellows (24) to be longitudinally displaceable.

14. Container according to any one of claims 1 to 13, **characterised in that** the sensor adapter (3, 3', 3" 3"', 3"") consists of a material resistant to beta radiation and/or to gamma radiation and/or to ETO.

15. Container according to any one of claims 1 to 14, **characterised in that** the container (2) is constructed as a single-use bioreactor (1).

## Revendications

1. Récipient (2) avec un adaptateur de capteur (3) pour la réception d'un agencement de capteur (10, 10''', 10"") pour la mesure au moins d'un paramètre de produits contenus dans un espace intérieur de récipient (7), l'adaptateur de capteur (3, 3', 3", 3"') étant agencé sur la paroi vers l'espace intérieur de récipient (7) et présente une ouverture de réception (9) accessible de l'extérieur d'un canal de réception (8, 8", 8"') délimité vers l'espace intérieur de récipient (7) pour l'adaptation de l'agencement de capteur (10, 10"', 10""),
**caractérisé en ce**
**que** le canal de réception (8, 8", 8"') de l'adaptateur de capteur (10) vers l'espace intérieur de contenant (7) présente au moins une surface limite formée par une membrane (13, 13', 13'', 13"', 13"", 14, 14', 14''), par laquelle le produit à mesurer ou les produits à mesurer peuvent être amenés à l'agencement de capteur (10, 10"', 10""), la membrane (13, 13', 13", 13"', 13"", 14, 14', 14") présentant une grosseur de pore entre 0,1 et 0,4 µm.

2. Récipient selon la revendication 1,
**caractérisé en ce**
**que** la membrane (13, 13', 13'', 13''', 13"", 14, 14', 14") présente une grosseur de pore ≤ 0,2 µm et forme une barrière stérile.

3. Récipient selon l'une quelconque des revendications 1 à 2,
**caractérisé en ce**
**que** la membrane (13, 13', 13", 13"', 14, 14', 14") présente des propriétés hydrophiles ou hydrophobes ou oléophobes.

4. Récipient selon l'une quelconque des revendications 1 à 2,
**caractérisé en ce**
**que** la membrane (13"") présente une ou plusieurs zones (22) avec des propriétés hydrophiles et une ou plusieurs zones (23) avec des propriétés hydrophobes.

5. Récipient selon l'une quelconque des revendications 1 à 4,
**caractérisé en ce**
**que** la membrane (13, 13', 13", 13"', 13"", 14, 14', 14") est réalisée en un polymère naturel.

6. Récipient selon la revendication 5,
**caractérisé en ce**
**que** la membrane (13, 13', 13", 13"', 13"", 14, 14', 14") est réalisée en acétate de cellulose ou en cellulose régénérée.

7. Récipient selon l'une quelconque des revendications 1 à 4,
**caractérisé en ce**
**que** la membrane (13, 13', 13'', 13''', 13"", 14, 14', 14") est réalisée en un polymère synthétique.

8. Récipient selon la revendication 7,
**caractérisé en ce**
**que** la membrane (13, 13', 13", 13"', 13"", 14, 14', 14") est réalisée en polysulfone.

9. Récipient selon l'une quelconque des revendications 1 à 8,
**caractérisé en ce**
**que** le canal de réception (8) forme une tige d'adaptateur (6) pénétrant dans l'espace intérieur de réacteur (7), laquelle présente deux membranes (13, 14) agencées l'une derrière l'autre avec différentes propriétés et dont la surface avant est fermée.

10. Récipient selon l'une quelconque des revendications 1 à 8,
**caractérisé en ce**
**que** le canal de réception (8') forme une tige d'adaptateur (6') pénétrant dans l'espace intérieur de réacteur (7) qui présente une première membrane (13') fermant sa surface avant et une seconde membrane (14') agencée sur sa périphérie avec différentes propriétés par rapport à la première membrane (13').

11. Récipient selon l'une quelconque des revendications 1 à 8,
**caractérisé en ce**
**que** le canal de réception (8") forme une tige d'adaptateur (6") pénétrant dans l'espace intérieur de réacteur (7) qui présente une première membrane (13") agencée sur sa périphérie, dont la surface avant est fermée et qui présente un canal latéral (19) menant vers l'extérieur, dont l'ouverture d'accès (20) se trouvant en dehors de l'espace intérieur de réacteur (7) est recouverte par une seconde membrane (14'').

12. Récipient selon l'une quelconque des revendications 1 à 11,
**caractérisé en ce**
**que** l'agencement de capteur (10") est agencé en forme de piston dans le canal de réception (8"') pour la génération d'une pression de manière mobile longitudinalement.

13. Récipient selon l'une quelconque des revendications 1 à 12,
**caractérisé en ce**
**que** l'agencement de capteur (10"") est agencé sur un soufflet (24) de manière mobile longitudinalement dans le canal de réception (8"").

14. Récipient selon l'une quelconque des revendications 1 à 13,
**caractérisé en ce**
**que** l'adaptateur de capteur (3, 3', 3", 3"', 3"") se compose d'un matériau résistant par rapport au rayonnement bêta et/ou rayonnement gamma et/ou ETO.

15. Récipient selon l'une quelconque des revendications 1 à 14,
**caractérisé en ce**
**que** le récipient (2) est réalisé comme un bioréacteur à usage unique (1).
